# FASCICULE DE BREVET EUROPEEN

(11) **EP 2 766 712 B1**
(45) Date de publication et mention de la délivrance du brevet: **23.09.2020**
(21) Numéro de dépôt: 12780227.0
(22) Date de dépôt: 08.10.2012
(51) Int. Cl.: G01N 21/64

(54) **PROCEDE DE DETECTION DE L'INTERACTION D'AU MOINS UNE ENTITE AVEC UNE COUCHE DIELECTRIQUE**
VERFAHREN ZUR ERKENNUNG DER INTERAKTION VON MINDESTENS EINER EINHEIT MIT EINER DIELEKTRISCHEN SCHICHT
METHOD FOR DETECTING THE INTERACTION OF AT LEAST ONE ENTITY WITH A DIELECTRIC LAYER

(30) Priorité: 11.10.2011 FR 1159174
(43) Date de publication de la demande: 20.08.2014
(73) Titulaire: Centre National de la Recherche Scientifique, 75794 Paris Cedex 16 (FR); INSERM - Institut National de la Santé et de la Recherche Médicale, 75654 Paris Cedex 13 (FR)
(72) Inventeur: NICHIPORUK, Tetyana, F-69780 Mions (FR); SERDIUK, Tetiana, F-69780 Mions (FR); LYSENKO, Volodymyr, F-69100 Villeurbanne (FR); ZAKHARKO, Yuriy, F-69003 Lyon (FR); GELOEN, Alain, F-69008 Lyon (FR); LEMITI, Mustapha, F-69006 Lyon (FR)
(74) Mandataire: IPAZ
(86) Numéro de dépôt international: PCT/FR2012/052274
(87) Numéro de publication internationale: WO 2013/054024

(56) Documents cités:
- US-A1- 2011 188 733
- T. SERDIUK ET AL: "Storage of luminescent nanoparticles in porous silicon: Toward a solid state "golden fleece"", MATERIALS LETTERS, vol. 65, no. 15-16, 1 août 2011 (2011-08-01), pages 2514-2517, XP055049014, ISSN: 0167-577X, DOI: 10.1016/j.matlet.2011.05.033
- PASCAL ANGER ET AL: "Enhancement and Quenching of Single-Molecule Fluorescence", PHYSICAL REVIEW LETTERS, vol. 96, no. 11, 1 mars 2006 (2006-03-01), XP055049030, ISSN: 0031-9007, DOI: 10.1103/PhysRevLett.96.113002
- F Giorgis ET AL: "Luminescence processes in amorphous hydrogenated silicon-nitride nanometric multilayers", Physical Review, vol. 60, no. 16 15 octobre 1999 (1999-10-15), pages 11572-11576, XP055026829, Extrait de l'Internet: URL:http://quantacomm.com/members/vinegoni /publications/prb_rc.pdf [extrait le 2012-05-10]
- PONS T ET AL: "On the quenching of semiconductor quantum dot photoluminescence by proximal gold nanoparticles", NANO LETTERS, ACS, US, vol. 7, no. 10, 1 octobre 2007 (2007-10-01), pages 3157-3164, XP007914595, ISSN: 1530-6984, DOI: 10.1021/NL071729+
- VALRIE DUPLAN ET AL: "A photoluminescence-based quantum semiconductor biosensor for rapid in situ detection of Escherichia coli", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 160, no. 1, 5 July 2011 (2011-07-05), pages 46-51, XP028110866, ISSN: 0925-4005, DOI: 10.1016/J.SNB.2011.07.010 [retrieved on 2011-07-19]
- VALÉRIE DUPLAN ET AL: "A photoluminescence-based quantum semiconductor biosensor for rapid in situ detection of Escherichia coli (Supporting Information)", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 160, no. 1, 1 December 2011 (2011-12-01), pages 46-51, XP055504018, NL ISSN: 0925-4005, DOI: 10.1016/j.snb.2011.07.010

## Description

La présente invention concerne le domaine technique de la détection de luminescence appliquée aux Biosciences. Plus précisément, la présente invention a pour objet un procédé de détection de l'interaction d'au moins une entité avec une couche diélectrique luminescente contenant différents niveaux électroniques. Un tel procédé est particulièrement adapté à l'étude de cellules vivantes ou d'organites cellulaires tels que les noyaux cellulaires et mitochondries.

Actuellement, l'imagerie fluorescente des cellules biologiques se fait en utilisant des fluorophores (http://fr.wikipedia.org/wiki/Fluorophore) et des nanoparticules (NPs) semi-conductrices II-VI et III-V (http://fr.wikipedia.org/wiki/Nanocristaux_de_semi-conducteurs). Les fluorophores utilisés sont souvent de nature organique et sont toxiques pour la plupart. De plus, ces molécules perdent leurs propriétés de luminescence en se dégradant sous l'effet de la lumière excitatrice, ce qui limite le temps d'observation à quelques minutes. Les NPs II-VI et III-V, quant à elles, présentent, par rapport aux fluorophores, les avantages suivants : (i) la longueur d'onde d'émission peut être contrôlée par leur taille, (ii) elles conduisent à des rendements de luminescence élevés et (iii) elles présentent une plus grande stabilité de l'émission. Cependant, ces NPs se sont révélées cytotoxiques, du fait de la libération d'ions toxiques (par exemple, Cd, Se...) sous photoexcitation.

La demande de brevet US2010/0035335 utilise des nanoparticules métalliques qui amplifient soit la luminescence naturelle des cellules, soit des marqueurs fluorescents intégrés à la cellule. Ce document se focalise donc soit sur l'amplification de l'autofluorescence cellulaire qui conduit à une seule couleur verte, soit sur celle des agents de coloration ajoutés aux cellules qui ont un effet cytotoxique. Le document "Luminescence processes in amorphous hydrogenated silicon-nitride nanometric multilayers" (F. Giorgis and C.F. Pirri, Physical Rview B, Vol. 60, Number 16) montre la détection de la photoluminescence dans une couche diélectrique de silicium.

Le document "A photoluminescence-based quantum semiconductor biosensor for rapid in situ détection of Escherichia coli" (Valerie Duplan et al., Sensors and Actuators B: Chemical), montre la détection des biomolecules basé sur la photoluminescence d'un sémi-conducteur GaAs.

Par conséquent, à l'heure actuelle, l'étude de biomolécules par détection de fluorescence nécessite de mettre en œuvre des biomolécules marquées ou conduit à des images en monocouleur qui correspondent à la la fluorescence naturelle, éventuellement amplifiée, des biomolécules dans certaines gammes de longueurs d'onde.

L'invention a justement pour objectif de proposer un nouveau procédé adapté à l'étude de différentes entités biologiques ou biomolécules, qui soit facile à mettre en œuvre et ne nécessite pas de marquage préalable des entités biologiques ou biomolécules.

Le procédé selon l'invention se doit donc d'être simple et compétitif, par rapport aux techniques antérieures précédemment rappelées.

Dans ce contexte, la présente invention concerne un procédé de détection de l'interaction d'au moins une entité avec une couche diélectrique contenant différents niveaux électroniques dans la bande d'énergie interdite de la couche diélectrique,
comprenant les étapes suivantes :
a) l'entité est déposée sur la couche diélectrique,
b) l'entité et la couche diélectrique sur laquelle elle est déposée sont soumises à une radiation électromagnétique excitatrice qui n'entraine pas de luminescence observable de l'entité elle-même, dans les conditions mises en œuvre à l'étape c), et
c) la luminescence de la couche diélectrique, dont les transitions électroniques radiatives et non-radiatives entre les niveaux énergétiques dans la bande interdite ont été influencées suite à son interaction avec l'entité, est détectée.

Plus particulièrement, dans le cadre de l'invention, l'entité est capable, par des interactions physico-chimiques avec la couche diélectrique sur laquelle elle est déposée, d'influencer les transitions électroniques radiatives et non-radiatives entre les niveaux énergétiques dans la bande interdite de la couche diélectrique provoquées par la radiation électromagnétique excitatrice externe utilisée.

Dans le procédé selon l'invention, c'est la luminescence de la couche diélectrique émise sous l'influence de l'entité (qui peut être en particulier une cellule vivante) qui est détectée. L'invention utilise la passivation de la couche diélectrique par la ou les entités déposées sur la couche diélectrique (qui peut être en particulier une cellule vivante) ce qui augmente le taux de recombinaisons radiatives de la couche, modifiant ainsi la luminescence émise. Le procédé selon l'invention n'utilise pas du tout l'effet plasmonique, comme c'est le cas dans le procédé décrit dans la demande de brevet US2010/0035335.

La description ci-dessous, en référence aux Figures annexées, permet de mieux comprendre l'invention.
La **Figure 1** est une vue schématique en coupe d'un substrat pouvant être utilisé dans le cadre de l'invention.
La **Figure 2** présente le diagramme des états énergétiques d'une couche diélectrique SiNₓₐ utilisée dans le cadre de l'invention, ainsi que certaines des transitions électroniques radiatives possibles.
La **Figure 3** est une photographie de dessus prise au microscope électronique à transmission (MET) d'une couche diélectrique SiNₓₐ avec xa = 0,5 déposée sur lame de verre obtenue conformément à l'exemple ci-après.
La **Figure 4** présente des photographies obtenues avec des fibroblastes 3T3 déposés sur support de verre (A) et support de verre recouvert d'une couche de SiNₓₐ (B) obtenue conformément à l'exemple ci-après.
La **Figure 5** présente des photographies obtenues avec des cellules épithéliales humaines saines (A) et des cellules épithéliales humaines cancéreuses (B) déposées sur support de verre recouvert d'une couche de SiNₐₓ obtenue conformément à l'exemple ci-après.
La **Figure 6** présente des photographies obtenues avec différentes cellules cancéreuses déposées sur support de verre recouvert d'une couche de SiNₐₓ obtenue conformément à l'exemple ci-après : (A) des cellules cancéreuses HUH7 et (B) des cellules cancéreuses sHSC.
La **Figure 7** présente des photographies obtenues, dans deux essais différents, avec des cellules cancéreuses sHSC déposées sur support de verre recouvert d'une couche de SiNₓₐ obtenue conformément à l'exemple ci-après.
La **Figure 8** présente des photographies obtenues, dans deux essais différents, avec des cellules épithéliales humaines saines déposées sur support de verre recouvert d'une couche de SiNₓₐ obtenue conformément à l'exemple ci-après.

Selon des modes de réalisation préférés, la couche diélectrique est composée :
- soit d'oxyde de silicium dans laquelle des nanoparticules de silicium sont réparties, la dite couche comportant des liaisons Si-H, Si-O-Si et Si-OH,
- soit de nitrure de silicium dans laquelle des nanoparticules de silicium sont réparties, la dite couche comportant des liaisons Si-H, Si-N-Si et N-H,
- soit d'un oxy-nitrure de silicium dans laquelle des nanoparticules de silicium sont réparties, la dite couche comportant des liaisons Si-H, Si-N-Si, Si-O-Si, Si-OH et N-H.

De manière particulièrement avantageuse, la couche diélectrique présente la stœchiométrie suivante en atomes de Si, N et O : SiOₓ avec 0<x<2, Si_{y}N_{z} avec 1<y<3 et 0<z<4 ou SiₜOᵤNᵥ avec 1<t<3, 0<u<1 et 0<v<2. Les stœchiométries données dans le cadre de l'invention incluent les nanoparticules de silicium présentes au sein de la couche.

En général, dans de telles couches, les nanoparticules de silicium représentent une fraction volumique de 5 à 75%, par rapport au volume total de la couche diélectrique (c'est-à-dire de la matrice + les nanoparticules de silicium). Cette fraction volumique sera fonction de la stœchiométrie de la couche. Les couches diélectriques plus riches en Si présenteront une fraction volumique de nanoparticules de Si plus importante. La présence des nanoparticules de Si peut être déterminée par MET, la stœchiométrie par les techniques de spectroscopie des éléments associées au MET, et la fraction volumique des particules de silicium par spectroscopie XPS.

Lorsque la couche diélectrique est composée d'oxyde de silicium dans laquelle des nanoparticules de silicium sont réparties, celle-ci comprend des liaisons Si-H, du fait de la passivation par l'hydrogène d'au moins une partie des défauts d'interface entre la matrice d'oxyde de silicium et les nanoparticules de silicium.

De même lorsque la couche diélectrique contenant différents niveaux électroniques dans la bande d'énergie interdite de la couche diélectrique est composée de nitrure de silicium ou d'oxy-nitrure de silicium dans laquelle des nanoparticules de silicium sont réparties, celle-ci comprend des liaisons Si-H et N-H, correspondant à une passivation par l'hydrogène d'au moins une partie des défauts d'interface entre la matrice de nitrure de silicium ou d'oxy-nitrure de silicium respectivement et les nanoparticules de silicium qu'elle contient.

De manière préférée, la couche diélectrique est une couche diélectrique de nitrure de silicium dans laquelle des nanoparticules de silicium sont réparties et qui est partiellement hydrogénée et dont la stœchiométrie en atomes de silicium et en atomes d'azote est SiNₓₐ avec xa allant de 0,4 à 0,8.

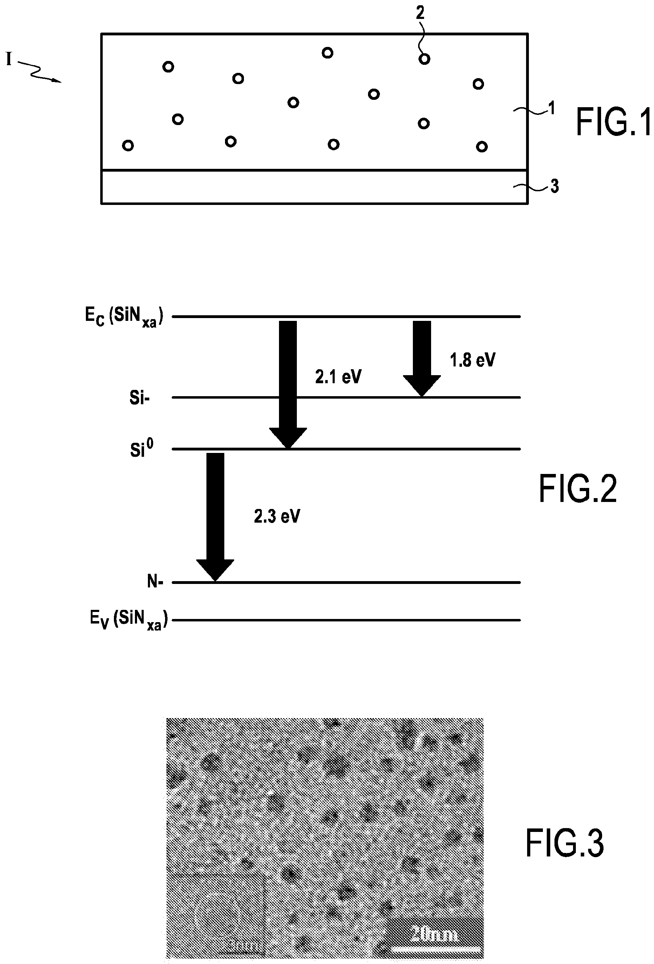

La **Figure 1** est une représentation schématique d'une couche diélectrique **I** constituée d'une matrice **1** dans laquelle des particules de silicium **2** sont réparties de manière homogène. La matrice **1** peut être :
- soit de l'oxyde de silicium comportant des liaisons Si-H, Si-O-Si et Si-OH,
- soit du nitrure de silicium comportant des liaisons Si-H, Si-N-Si et N-H,
- soit un oxy-nitrure de silicium comportant des liaisons Si-H, Si-N-Si, Si-O-Si, Si-OH et N-H.

La **Figure 2** présente le diagramme des états énergétiques d'une couche diélectrique SiNₓₐ telle que définie ci-dessus, ainsi que certaines des transitions électroniques radiatives possibles entre les niveaux énergétiques dans la bande interdite. Conformément à certaines données reportées dans la littérature (Robertson J. et al., Appl. Phys. Lett. 1984, 44, 415-417 et Mo C. et al. J. Appl. Phys. 1993, 73, 5185-5188), la première transition électronique (Ec(SiNₓₐ)->Si⁻) correspond à une émission rouge relative à la transition entre la bande de conduction (Ec) de la couche SiNₓₐ et les états de défaut Si⁻ des nanoparticules de silicium. La deuxième (Ec(SiNₓₐ)->Si⁰ correspondant à une émission jaune) intervient entre le niveau Ec de la matrice diélectrique et les atomes de silicium neutres des nanoparticules de silicium. La troisième voie de recombinaison (donnant lieu à une émission verte) correspond à la transition électronique (Si⁰ -> N⁻) qui se produit entre les atomes de silicium neutres des nanoparticules de silicium et les états de défaut N⁻ de la matrice diélectrique. Il existe également d'autres voies de recombinaison dans le spectre d'émission bleu pour ce type de couche (Robertson J. et al., Appl. Phys. Lett. 1984, 44, 415-417 et Mo C. et al. J. Appl. Phys. 1993, 73, 5185-5188).

Il est également possible d'utiliser d'autres couches diélectriques notamment du type organique, par exemple les polymères, notamment utilisés dans le domaine de la photovoltaïque.

L'interaction de l'entité avec la couche diélectrique permet d'atteindre de tels niveaux de transition et conduit donc à une émission de luminescence. Dans le cas de cellules vivantes notamment, l'émission pourra être différente en fonction de la zone de la cellule en contact avec la couche diélectrique, comme cela apparaîtra, par la suite en référence aux Figures.

Le plus souvent, l'entité sera déposée sur la couche diélectrique et laisser pendant un temps suffisant pour obtenir une interaction de l'entité avec la couche diélectrique entrainant des interactions physico-chimiques avec la couche diélectrique sur laquelle elle est déposée, qui vont influencer les transitions électroniques radiatives et non-radiatives entre les niveaux énergétiques dans la bande interdite provoquées par une radiation électromagnétique excitatrice externe. Ainsi, l'émission de luminescence obtenue diffère de celle qui serait obtenue si l'entité était déposée sur un substrat classique, telle qu'une lame de verre, ainsi que de celle obtenue avec la couche diélectrique seule.

Les nanoparticules de silicium présentes dans la couche diélectrique peuvent se présenter sous la forme de particules essentiellement sphériques, de bâtonnets ou de particules de forme irrégulière. En général, les particules seront essentiellement sphériques. De manière préférée, les nanoparticules de silicium présentent une taille inférieure à 50 nm, et avantageusement une taille appartenant à la gamme allant de 1 à 20 nm, et préférentiellement à la gamme allant de 1 à 7 nm. Le plus souvent, les nanoparticules utilisées sont essentiellement sphériques, c'est-à-dire que leur forme ne dévie pas de plus de 10% d'une sphère parfaite. La taille d'une nanoparticule correspond à son diamètre dans le cas de particules sphériques ou à son diamètre équivalent dans le cas de particules non sphériques. La mesure du diamètre équivalent d'une nanoparticule peut être réalisée en mesurant la surface de chaque nanoparticule sur un cliché par microscopie électronique à transmission. Chaque nanoparticule est assimilée à une sphère parfaite et le diamètre équivalent est calculé à partir de la surface (S) d'analyse observée (section transverse) à partir de la formule S=ΠR² avec R qui correspond au rayon de la sphère parfaite, et donc de la section transverse, à laquelle est assimilée la nanoparticule.

De manière préférée, la couche diélectrique présente une épaisseur inférieure à 500 nm, et typiquement appartenant à la gamme allant de 30 à 200 nm et préférentiellement à la gamme allant de 50 à 150 nm, qui sera bien entendu en relation avec la taille des nanoparticules de silicium présentes.

La couche diélectrique est en général déposée sur un substrat **3** jouant le rôle de support, comme illustré **Figure 1****.** En général, de tels substrats seront de type lame ou lamelle, en verre ou en quartz, ou encore en silicium ou plus généralement tout support qui supporterait le procédé de dépôt de la couche diélectrique.

Les couches diélectriques peuvent être obtenues dans le cadre de l'invention par un procédé de dépôt chimique en phase vapeur (CVD), et en particulier par un procédé de dépôt chimique en phase vapeur assisté par plasma nommé PECVD.

Un dépôt CVD est réalisé par réaction des différents constituants d'une phase vapeur. Ces constituants sont créés par la dissociation de plusieurs espèces. Ces réactions nécessitent un "moteur énergétique" qui peut être l'énergie thermique, mais aussi l'énergie fournie par plasma ou une autre source énergétique.

Dans le cas où la matrice est en oxyde de silicium, le dépôt sera effectué à partir d'un mélange gazeux composé de silane (SiH₄) et de protoxyde d'azote (N₂O), éventuellement dans un gaz vecteur du type argon, hélium ou hydrogène. Dans le cas où la matrice est en nitrure de silicium, le dépôt sera effectué à partir d'un mélange gazeux contenant SiH₄ et de l'ammoniaque (NH₃) ou de l'azote (N₂), jouant le rôle de gaz précurseurs. Dans le cas où la matrice est en un oxy-nitrure de silicium, le dépôt sera effectué à partir d'un mélange gazeux composé de SiH₄, NH₃ (ou N₂) et N₂O.

Les différentes stœchiométries sont obtenues en faisant varier le ratio des gaz précurseurs. Le temps de dépôt détermine une épaisseur différente.

Les couches déposées par CVD contiennent directement des nanoparticules de silicium intégrées dans leur masse. Leur taille et la densité des nanoparticules de Si dépendent principalement de la stœchiométrie des couches. Par exemple, dans le cas d'une matrice en nitrure de silicium, plus les couches sont riches en Si, plus la taille des nanoparticules est grande. Plus les couches sont riches en azote, plus la taille des nanoparticules est petite et leur densité est faible. A titre indicatif, pour une matrice de SiNₓₐ avec xa=0,4-0,8, le plus souvent, la taille des nanoparticules de silicium appartiendra à la gamme allant de 1 à 7 nm, et plus précisément seront de l'ordre de 3 à 4 nm.

Dans le cas de la PECVD, de tels dépôts pourront être effectués à une température appartenant à la gamme allant de 50 à 500 °C, et typiquement de l'ordre de 370°C. La pression, la fréquence et la puissance injectée pour générer le plasma, ainsi que les débits gazeux dépendront du réacteur utilisé et seront adaptés par l'homme du métier.

Les couches déposées obtenues contiennent des nanoparticules de Si intégrées dans la matrice d'oxyde de silicium, de nitrure de silicium ou d'oxy-nitrure de silicium, en fonction du mélange gazeux utilisé. De telles couches sont partiellement hydrogénées, c'est pourquoi l'on mentionne la présence de liaisons Si-H et Si-OH, dans le cas de l'oxyde de silicium, la présence de liaisons Si-H et N-H, dans le cas du nitrure de silicium et la présence de liaisons Si-H, Si-OH et N-H, dans le cas de l'oxy-nitrure de silicium. La préparation de telles couches diélectriques est notamment décrite par J-F Lelievre, dans "Elaboration de SiNx:H par PECVD: optimisation des propriétés optiques, passivantes et structurales pour applications photovoltaïques", Thèse INSA de Lyon, 2007, p. 187 et par J. Dupuis, dans "Elaboration et charactérisation de couches SiOxNy:H et SiNx:H réalisées par méthode PECVD: application à la face arrière des cellules photovoltaïques en Silicium", Thèse INSA de Lyon, 2009, p.161, auxquelles on pourra se référer pour plus de détails.

Il est également possible que le dépôt soit réalisé par d'autres techniques de dépôt chimique en phase vapeur bien connues de l'homme de l'art, à savoir la technique de dépôt chimique en phase vapeur assistée par photon UV UVCVD (pour « UV(Hg) photon assisted Chemical Vapor Déposition »), la technique de dépôt chimique en phase vapeur à pression atmosphérique APCVD (pour « Atmospheric Chemical Vapor Déposition ») ou la technique de dépôt chimique en phase vapeur à basse pression LPCVD (pour « Low Pressure Chemical Vapor Déposition »). Pour plus de détails sur de telles techniques, on pourra se référer à G. S. May, S.M. Sze, Fundamentals of semiconductor fabrication; 2008, Wiley-Interscience, ISBN-0471-23279-3.

Le procédé selon l'invention peut être appliqué à tout type d'entité biologique, notamment aux molécules rentrant dans la composition des cellules, telles que les protéines, les lipides (phospholipides ou glycolipides), les ADN, les ARN, ou encore aux cellules ou plus généralement aux organites cellulaires tels que les noyaux et les mitochondries. Le procédé selon l'invention peut être utilisé sur tout type de substances (produits dans le domaine biomédical, agro-alimentaire, cosmétique, la parfumerie, etc.) qui peuvent avoir une interaction physico-chimique avec la couche diélectrique et ainsi influencer les transitions électroniques radiatives et non-radiatives entre les niveaux énergétiques dans la bande interdite provoquées par une radiation électromagnétique excitatrice externe. Ces entités une fois déposées sur la couche vont influencer l'émission de luminescence obtenue suite à une excitation électromagnétique, tel qu'un rayonnement lumineux, et conduire à l'émission d'un jeu de couleurs différentes par les couches diélectriques utilisées, et notamment les couches d'oxyde de silicium, de nitrure de silicium ou d'oxy-nitrure de silicium telles que définies dans le cadre de l'invention dans lesquelles des nanoparticules de Si sont intégrées.

Il est possible de déposer une entité unique sur la couche diélectrique (notamment une seule cellule) ou une population d'entités (notamment une culture cellulaire). L'entité pourra être déposée, sous une forme en solution ou en suspension dans un solvant approprié, ou telle quelle. En général, le temps de contact entre l'entité et la couche diélectrique, avant mesure de la luminescence, et éventuellement excitation si l'excitation ne se fait pas grâce à la lumière naturelle, sera de préférence supérieur à 10 s, voire à 1 heure ou 1 jour, ce temps étant fonction de l'entité étudiée. Par exemple, pour certains types de cellules, des temps plus longs de un à plusieurs jours pourront être nécessaires pour qu'une image multi-couleurs notamment apparaisse.

Le procédé selon l'invention est particulièrement avantageux dans le cas où l'entité est une cellule vivante. Dans ce cas, la cellule vivante pourra être déposée et être mise en croissance directement sur la couche diélectrique. Pour cela, tout milieu de culture bien connu de l'homme du métier pourra être utilisé. De préférence, un rinçage pourra être effectué avant mesure de la luminescence, voire avant excitation si l'excitation ne se fait pas grâce à la lumière naturelle. Avant la détection, il pourra être utile de fixer la ou les cellules selon des techniques bien connues de l'homme du métier, par exemple, grâce à un rinçage à l'éthanol.

Le procédé selon l'invention, en particulier lorsque l'entité est une cellule ou un noyau cellulaire ou une mitochondrie, pourra être utilisé pour effectuer de la différentiation cellulaire, pour diagnostiquer une pathologie, et en particulier un cancer ou encore pour évaluer l'efficacité d'un traitement. Dans ces différents cas, on opèrera généralement par comparaison de la luminescence émise par la cellule ou l'organite cellulaire à étudier avec une luminescence de référence : par exemple une cellule ou organite sain, dans le cas du diagnostic d'une pathologie, ou une cellule ou organite avant traitement, dans le cas de l'évaluation de l'efficacité d'un traitement. Du fait de modifications intervenues au sein de la cellule ou de l'organite cellulaire, son interaction avec la couche diélectrique va être différente, conduisant sous excitation électromagnétique, à une émission de luminescence différente.

Plus généralement, on peut citer les applications suivantes pour le procédé selon l'invention :
- la visualisation en multi-couleurs des cellules biologiques, sans addition d'agents fluorescents spécifiques,
- l'identification des cellules, et notamment la distinction entre cellules saines et cellules cancéreuses,
- La détection de l'état métabolique (notamment la distinction entre phase de mitose, état d'apoptose, ...) d'une culture cellulaire, ou de certaines cellules dans une population de cellules.

Quel que soit le mode de réalisation de l'invention, il peut être prévu que la couche diélectrique ne comporte pas, ni dans sa masse, ni en surface, de particules métalliques. Néanmoins, bien que ces variantes ne soient pas préférées, il est possible d'intégrer, dans ou en surface de la couche diélectrique, des nanoparticules d'argent, par exemple. Pour la préparation de tels supports, on pourra, par exemple, se référer à Solar Energy Materials and Solar Cells, Volume 94, Issue 12, December 2010, Pages 2314-2317.

Par ailleurs, quel que soit le mode de réalisation de l'invention, la détection peut être réalisée sans ajout d'agent luminescent à l'entité. C'est-à-dire, notamment, sans marquage préalable de l'entité.

Dans le cadre de l'invention, la radiation électromagnétique excitatrice est, le plus souvent, un rayonnement de lumière visible à l'œil humain, un rayonnement Infra Rouge, Ultraviolet ou Rayon X. De façon avantageuse, il est possible d'utiliser une radiation électromagnétique dans la gamme de longueurs d'onde allant de 250 à 700 nm.

Le moyen de détection de la luminescence peut être un capteur CCD (de l'anglais « Charge-Coupled Device », pour dispositif à transfert de charge) relié à un système informatique de traitement de l'image obtenue. La détection, quant à elle, sera de préférence réalisée dans la gamme de longueurs d'onde allant de 300 à 2000 nm.

De manière avantageuse, il est possible, avec le procédé selon l'invention que la détection de la luminescence de la couche diélectrique soit obtenue à l'étape c) sous la forme d'une image en plusieurs couleurs.

L'utilisation de l'invention est possible en biologie ou en médecine et consistera à tout simplement remplacer les substrats constitués en verre classiquement utilisés comme supports des cultures cellulaires (lors de leurs observation en microscopie optique) par des substrats, notamment de verre, porteurs d'une couche diélectrique et notamment d'une couche d'oxyde de silicium, de nitrure de silicium ou d'oxy-nitrure de silicium telle que définie dans le cadre de l'invention dans laquelle des nanoparticules de Si sont intégrées.

Le procédé selon l'invention offre de nouvelles approches dans le domaine de l'imagerie cellulaire, et permet notamment la visualisation en multi-couleurs de cellules (ou plus généralement d'entités biologiques), sans nécessiter de mettre en oeuvre un quelconque agent fluorescent tels que les fluorophores ou les nanoparticules fluorescentes qui perturbent ou changent complètement le fonctionnement normal des cellules ou des organites cellulaires.

Le procédé selon l'invention permet une visualisation efficace *in-vitro* d'entité biologique, pour des applications diverses en biologie ou médecine. Le procédé selon l'invention est particulièrement adapté à la visualisation et à l'étude de cellules, notamment sous la forme de culture cellulaire, ou encore d'organites cellulaires tels que des noyaux ou mitochondries qui peuvent être isolés à partir de cellules.

Les exemples ci-après permettent d'illustrer l'invention mais n'ont aucun caractère limitatif.

### Préparation des substrats

Les substrats constitués d'une lame de verre recouverte d'une couche diélectrique de nitrure de silicium partiellement hydrogénée dans laquelle des nanoparticules de silicium sont réparties, nommées dans la suite « SiNₓₐ/verre », ont été réalisés par dépôt PECVD dans un réacteur semi-industriel fonctionnant à basse fréquence (440kHz). Un mélange gazeux composé de silane (SiH₄) et d'ammoniaque (NH₃) est introduit dans le réacteur avec un débit de 800sccm. La pression et la puissance injectée sont fixées à respectivement 1,5 Torr et 0,26 W/cm². Les dépôts sont effectués à une température de 370°C. La vitesse de dépôt est environ de 25nm/min pour la couche de SiNₓₐ, avec xa=0,5. Le temps de dépôt est ajusté pour obtenir des épaisseurs de 60, 100, 150 et 200 nm.

Le ratio des débits de gaz ammoniaque/silane dans le gaz circulant détermine la stœchiométrie de la couche diélectrique déposée (nanoparticules de silicium comprises) qui est donc égale à 5. Le dépôt présente une épaisseur de 60 à 200 nm, en fonction des temps de dépôt. La **Figure 3** est une photographie TEM du dépôt obtenu et met en évidence la présence de particules de silicium de diamètre compris entre 2 et 4 nm au sein de la matrice de nitrure de silicium.

### Détection de la fluorescence

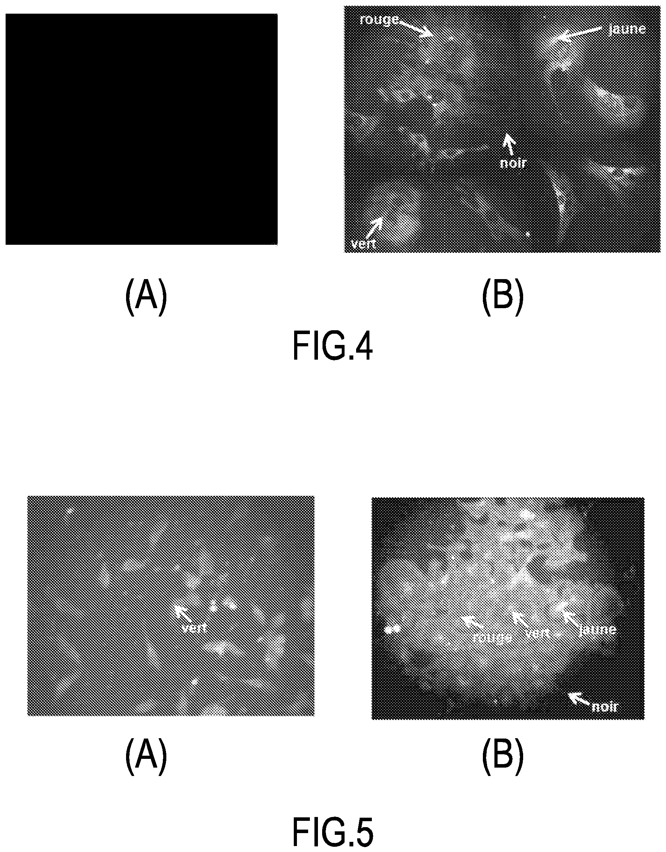

Toutes les photographies présentées sur les **Figures 4** à **8** ont été obtenues avec un microscope à fluorescence (Leica DMI 4000B) avec une combinaison de filtres permettant une excitation dans l'UV/violet (2,7-3,5 eV) avec une gamme spectrale d'observation inférieure à 2,64 eV. Il a été constaté que les couleurs ne changent pas si la stœchiométrie reste la même et les épaisseurs augmentent. Les Figures présentées ont été réalisées avec une couche d'épaisseur de 60 nm telle que réalisée précédemment. Il a également été constaté qu'avec une stœchiométrie obtenue avec un ratio de débits de gaz ammoniaque/silane de 3, des résultats comparables ont été obtenus.

Des cultures cellulaires de fibroblastes 3T3-L1 (American Type Culture Collection, Manassas, VA, USA) ont été réalisées sur des substrats SiN_{Xa}/verre tels que précédemment obtenus dans un milieu Eagle's modifié Dulbecco's (DMEM) supplémenté avec 10% de sérum de veau nouveau-né, 4 mM de glutamine, 4 nM d'insuline (Actrapid Human; Novo), 10 mM d'Hepes, 25 µg d'ascorbate de sodium, 100 IU de pénicilline, 100 µg de streptomycine, et 0,25 mg/l d'amphotéricine B à 37°C dans une atmosphère saturée en eau avec 5% de CO₂ dans l'air, dans un incubateur Heraeus (BB16). Après la période d'incubation et avant l'observation au microscope, les cellules ont été rincées deux fois avec de l'éthanol pur et ont été fixées sur les substrats.

Les images présentées **Figure 4** montrent l'efficacité du procédé selon l'invention pour la visualisation fluorescente des cellules biologiques sans aucun agent spécifique complémentaire. Les deux images ont été obtenues dans les mêmes conditions d'acquisition optique. On voit **(****Figure 4(A)****)** que la fluorescence naturelle (de couleur verte) des cellules 3T3-L1 fixées sur des supports ordinaires à base du verre est inexistante. Au contraire, les mêmes cellules étalées sur les substrats tels qu'obtenus précédemment donnent une fluorescence bien visible **(****Figure 4(B)**) avec une image en multi-couleurs avec des zones de rouge/jaune et vert. Sur la **Figure 4(B)****,** les endroits où on ne voit pas de cellules correspondent à la couche SiNₓₐ en l'absence de cellules. Aux mêmes conditions expérimentales, l'intensité de luminescence des couches toutes seules est donc très faible, ce qui prouve que les cellules déposées non seulement donnent un jeu de couleurs donné, mais également passivent les états non-radiatifs.

Les couleurs obtenues correspondent bien aux transitions possibles telles qu'illustrées **Figure 2****.** En plus, la cellule influe différemment sur le substrat, de sorte qu'il est possible de distinguer les diverses compartiments de la cellule qui vont correspondre à des émissions de fluorescence de couleurs différentes. Il est ainsi possible de distinguer le noyau, le réticulum endoplasmique et le cytoplasme. De plus, l'intensité de fluorescence détectée est bien supérieure à celle obtenue avec le signal d'auto-fluorescence des cellules (vert uniquement) obtenu dans les mêmes conditions d'acquisition en termes de temps d'acquisition et d'intensité d'excitation. Ces résultats montrent qu'il est possible de détecter des cellules, avec le procédé selon l'invention, sans fluorophore, ni nanoparticule luminescente additionnels.

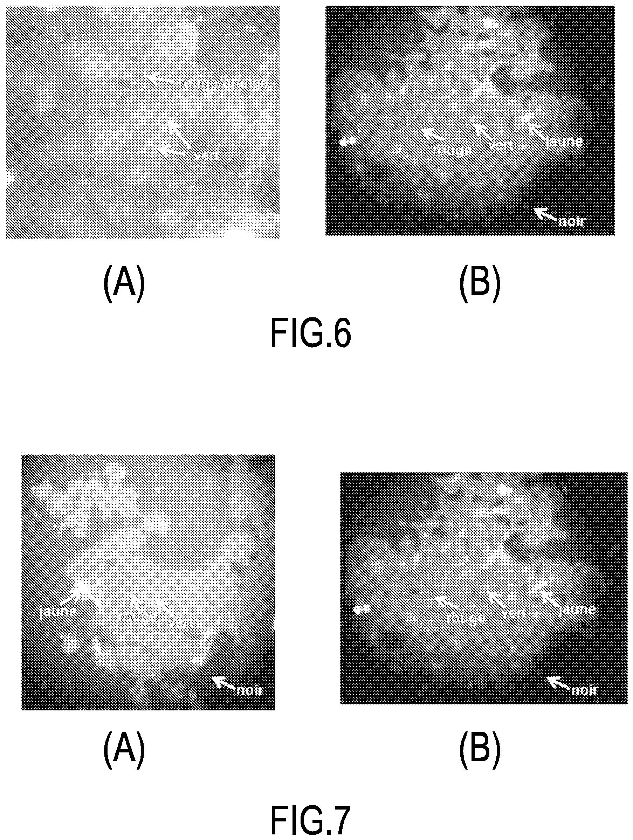

Les **Figures 5** et **6** mettent en évidence la fluorescence différente obtenue avec des cellules différentes, de par la nature même des cellules **(****Figure 6****)** ou de par leur caractère sain *versus* tumoral **(****Figure 5****).**

Quand elles sont mises en culture sur le même support SiNₓₐ/verre tel qu'obtenu précédemment, on observe une différence très marquée entre les cellules saines et les cellules cancéreuses **(****Figure 5****).** La nature des cellules étant différentes, l'interaction avec la couche diélectrique est différente, ce qui conduit à une émission de fluorescence différente, permettant de différencier les deux lignées cellulaires qui sont pourtant du même type (cellules épithéliales). La seule différence est la tumorigénicité, (A) cellules épithéliales humaines immortalisées, (B) cellules cancéreuses épithéliales HSC.

De la même manière, il est possible de différencier des cellules cancéreuses de nature différentes comme le montre la **Figure 6****.**

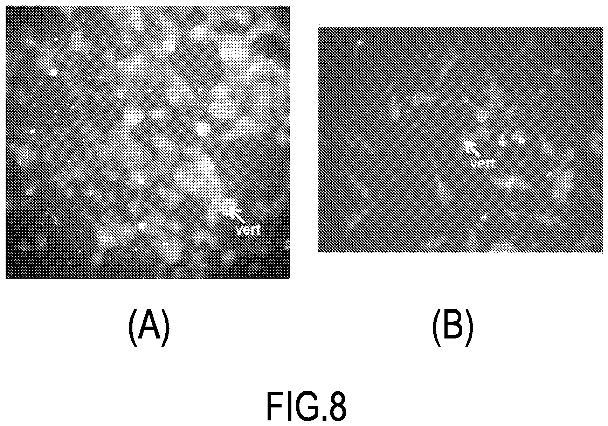

Les **Figures 7** et **8** mettent en évidence la reproductibité des photographies obtenues, dans deux essais différents réalisés avec les mêmes cellules et le même support recouvert d'une couche de SiNₓₐ/verre tel qu'obtenu précédemment.

## Revendications

1. Procédé de détection de l'interaction d'au moins une entité avec une couche diélectrique contenant différents niveaux électroniques dans la bande d'énergie interdite de la couche diélectrique,
dans lequel :
a) l'entité est déposée sur la couche diélectrique,
b) l'entité et la couche diélectrique sur laquelle elle est déposée sont soumises à une radiation électromagnétique excitatrice qui n'entraine pas de luminescence observable de l'entité elle-même, dans les conditions mises en œuvre à l'étape c) et
c) la luminescence de la couche diélectrique, dont les transitions électroniques radiatives et non-radiatives entre les niveaux énergétiques dans la bande interdite ont été influencées suite à son interaction avec l'entité, est détectée.

2. Procédé selon la revendication 1 **caractérisé en ce que** le couche diélectrique est composée :
- soit d'oxyde de silicium dans laquelle des nanoparticules de silicium sont réparties, la dite couche comportant des liaisons Si-H, Si-O-Si et Si-OH,
- soit de nitrure de silicium dans laquelle des nanoparticules de silicium sont réparties, la dite couche comportant des liaisons Si-H, Si-N-Si et N-H,
- soit d'un oxy-nitrure de silicium dans laquelle des nanoparticules de silicium sont réparties, la dite couche comportant des liaisons Si-H, Si-N-Si, Si-O-Si, Si-OH et N-H,

3. Procédé selon l'une des revendications précédentes **caractérisé en ce que** l'entité est capable, par des interactions physico-chimiques avec la couche diélectrique sur laquelle elle est déposée, d'influencer les transitions électroniques radiatives et non-radiatives entre les niveaux énergétiques dans la bande interdite provoquées par la radiation électromagnétique excitatrice externe.

4. Procédé selon l'une des revendications précédentes **caractérisé en ce que** la couche diélectrique présente la stœchiométrie suivante en atomes de Si, N et O :
SiOₓ avec 0 < x < 2, Si_{y}N_{z} avec 1 < y < 3 et 0 < z < 4 ou SiₜOᵤNᵥ avec 1 < t < 3, 0 < u < 1 et 0 < v < 2.

5. Procédé selon l'une des revendications précédentes **caractérisé en ce que** les nanoparticules de silicium présentent une taille appartenant à la gamme allant de 1 à 20 nm, de préférence à la gamme allant de 1 à 7 nm.

6. Procédé selon l'une des revendications précédentes **caractérisé en ce que** la couche diélectrique présente une épaisseur inférieure à 500 nm, et de préférence de 30 à 200 nm, et préférentiellement de 50 à 150 nm.

7. Procédé selon l'une des revendications précédentes **caractérisé en ce que** la couche diélectrique est une couche diélectrique de nitrure de silicium dans laquelle des nanoparticules de silicium sont réparties et qui est partiellement hydrogénée et dont la stœchiométrie en atomes de silicium et en atomes d'azote est SiNₓₐ avec xa allant de 0,4 à 0,8.

8. Procédé selon l'une des revendications précédentes **caractérisé en ce que** la couche diélectrique ne comporte pas, ni dans sa masse, ni en surface, des particules métalliques.

9. Procédé selon l'une des revendications précédentes **caractérisé en ce que** la radiation électromagnétique excitatrice est un rayonnement de lumière visible à l'œil humain, un rayonnement Infra Rouge, Ultraviolet ou Rayon X.

10. Procédé selon l'une des revendications précédentes **caractérisé en ce que** la détection de la luminescence de la couche diélectrique est réalisée à l'étape c) sous la forme d'une image en plusieurs couleurs.

11. Procédé selon l'une des revendications précédentes **caractérisé en ce que** la couche diélectrique est obtenue par un procédé de dépôt chimique en phase vapeur assisté par plasma nommé PECVD.

12. Procédé selon l'une des revendications précédentes **caractérisé en ce que** la détection est réalisée sans ajout d'agent luminescent à l'entité.

13. Procédé selon l'une des revendications précédentes **caractérisé en ce que** l'entité est une entité biologique, notamment une molécule rentrant dans la composition des cellules, telle qu'une protéine, un lipide, un ADN, un ARN, une cellule ou un organite cellulaire tel qu'un noyau ou une mitochondrie.

14. Procédé selon la revendication 13 **caractérisé en ce que** l'entité est un organite cellulaire tel qu'un noyau ou une mitochondrie, ou de préférence, l'entité est une cellule vivante.

15. Procédé selon la revendication 14 **caractérisé en ce que** l'entité est une cellule vivante qui est déposée et mise en croissance sur la couche diélectrique.

16. Utilisation d'un procédé selon l'une des revendications 13 à 15 pour effectuer de la différentiation cellulaire.

17. Utilisation d'un procédé selon l'une des revendications 13 à 15 pour diagnostiquer une pathologie, et en particulier un cancer.

18. Utilisation d'un procédé selon l'une des revendications 13 à 15 pour évaluer l'efficacité d'un traitement.

## Patentansprüche

1. Verfahren zum Erfassen der Interaktion von wenigstens einer Einheit mit einer dielektrischen Schicht, die unterschiedliche elektronische Niveaus in dem verbotenen Energieband der dielektrischen Schicht enthält,
bei dem:
a) die Einheit auf der dielektrischen Schicht abgeschieden wird,
b) die Einheit und die dielektrische Schicht, auf der sie abgeschieden ist, einer elektromagnetischen Erregerstrahlung ausgesetzt werden, die unter den in Schritt c) umgesetzten Bedingungen zu keiner beobachtbaren Lumineszenz der Einheit selbst führt, und
c) die Lumineszenz der dielektrischen Schicht, deren elektronische Strahlungs- und Nichtstrahlungsübergänge zwischen den Energieniveaus in dem verbotenen Band infolge ihrer Interaktion mit der Einheit beeinflusst worden sind, erfasst wird.

2. Verfahren nach Anspruch 1, **dadurch gekennzeichnet, dass** die dielektrische Schicht zusammengesetzt ist:
- entweder aus Siliziumoxid, in dem Siliziumnanopartikel verteilt sind, wobei die Schicht Si-H-, Si-O-Si- und Si-OH-Bindungen umfasst,
- oder aus Siliziumnitrid, in dem Siliziumnanopartikel verteilt sind, wobei die Schicht Si-H-, Si-N-Si- und N-H-Bindungen umfasst,
- oder aus einem Siliziumoxynitrid, in dem Siliziumnanopartikel verteilt sind, wobei die Schicht Si-H-, Si-N-Si-, Si-O-Si-, Si-OH- und N-H-Bindungen umfasst.

3. Verfahren nach einem der vorhergehenden Ansprüche, **dadurch gekennzeichnet, dass** die Einheit durch physikalisch-chemische Interaktionen mit der dielektrischen Schicht, auf der sie abgeschieden ist, in der Lage ist, die elektronischen Strahlungs- und Nichtstrahlungsübergänge zwischen den Energieniveaus in dem verbotenen Band, welche durch die externe elektromagnetische Erregerstrahlung bewirkt werden, zu beeinflussen.

4. Verfahren nach einem der vorhergehenden Ansprüche, **dadurch gekennzeichnet, dass** die dielektrische Schicht die folgende Stöchiometrie an Si-, N- und O-Atomen aufweist:
SiOₓ wobei 0 < x < 2, Si_{y}N_{z} wobei 1 < y < 3 und 0 < z < 4 oder SiₜOᵤNᵥ wobei 1 < t < 3, 0 < u < 1 und 0 < v < 2.

5. Verfahren nach einem der vorhergehenden Ansprüche, **dadurch gekennzeichnet, dass** die Siliziumnanopartikel eine zu dem Bereich von 1 bis 20 nm, vorzugsweise zu dem Bereich von 1 bis 7 nm gehörende Größe aufweisen.

6. Verfahren nach einem der vorhergehenden Ansprüche, **dadurch gekennzeichnet, dass** die dielektrische Schicht eine Dicke von weniger als 500 nm und vorzugsweise von 30 bis 200 nm und bevorzugt von 50 bis 150 nm aufweist.

7. Verfahren nach einem der vorhergehenden Ansprüche, **dadurch gekennzeichnet, dass** die dielektrische Schicht eine dielektrische Schicht aus Siliziumnitrid ist, in der Siliziumnanopartikel verteilt sind und die teilweise hydriert ist und deren Stöchiometrie an Siliziumatomen und an Stickstoffatomen SiNₓₐ ist, wobei xa im Bereich von 0,4 bis 0,8 liegt.

8. Verfahren nach einem der vorhergehenden Ansprüche, **dadurch gekennzeichnet, dass** die dielektrische Schicht weder in ihrer Masse noch auf der Oberfläche Metallpartikel enthält.

9. Verfahren nach einem der vorhergehenden Ansprüche, **dadurch gekennzeichnet, dass** die elektromagnetische Erregerstrahlung eine für das menschliche Auge sichtbare Lichtstrahlung, eine Infrarot-, Ultraviolett- oder Röntgenstrahlung ist.

10. Verfahren nach einem der vorhergehenden Ansprüche, **dadurch gekennzeichnet, dass** die Erfassung der Lumineszenz der dielektrischen Schicht in Schritt c) in Form eines Mehrfarbenbildes erfolgt.

11. Verfahren nach einem der vorhergehenden Ansprüche, **dadurch gekennzeichnet, dass** die dielektrische Schicht durch ein Verfahren der plasmaunterstützten chemischen Gasphasenabscheidung, welches als PECVD bezeichnet wird, erhalten wird.

12. Verfahren nach einem der vorhergehenden Ansprüche, **dadurch gekennzeichnet, dass** die Erfassung ohne Zugabe eines Lumineszenzmittels zu der Einheit durchgeführt wird.

13. Verfahren nach einem der vorhergehenden Ansprüche, **dadurch gekennzeichnet, dass** die Einheit eine biologische Einheit ist, insbesondere ein Molekül, das Bestandteil von Zellen ist, wie ein Protein, ein Lipid, eine DNA, eine RNA, eine Zelle oder ein zelluläres Organell, wie ein Kern oder ein Mitochondrium.

14. Verfahren nach Anspruch 13, **dadurch gekennzeichnet, dass** die Einheit ein zelluläres Organell, wie ein Kern oder ein Mitochondrium, ist oder vorzugsweise die Einheit eine lebende Zelle ist.

15. Verfahren nach Anspruch 14, **dadurch gekennzeichnet, dass** die Einheit eine lebende Zelle ist, die auf der dielektrischen Schicht abgeschieden und in Wachstum versetzt wird.

16. Verwendung eines Verfahrens nach einem der Ansprüche 13 bis 15, zur Durchführung der Zelldifferenzierung.

17. Verwendung eines Verfahrens nach einem der Ansprüche 13 bis 15, zur Diagnose einer Pathologie und insbesondere von Krebs.

18. Verwendung eines Verfahrens nach einem der Ansprüche 13 bis 15, zur Bewertung der Wirksamkeit einer Behandlung.

## Claims

1. Method for detecting the interaction of at least one entity with a dielectric layer containing different electron levels in the energy band gap of the dielectric layer,
in which:
a) the entity is deposited on the dielectric layer,
b) the entity and the dielectric layer on which it is deposited are subjected to exciting electromagnetic radiation which does not give rise to observable luminescence in the entity itself, under the conditions implemented in step c) and
c) the luminescence of the dielectric layer, in which the radiative and non-radiative electron transitions between the energy levels in the band gap have been influenced following its interaction with the entity, is detected.

2. Method according to claim 1 **characterized in that** the dielectric layer is composed of:
- either silicon oxide in which silicon nanoparticles are distributed, said layer comprising Si-H, Si-O-Si and Si-OH bonds,
- or silicon nitride in which silicon nanoparticles are distributed, said layer comprising Si-H, Si-N-Si and N-H bonds,
- or a silicon oxynitride in which silicon nanoparticles are distributed, said layer comprising Si-H, Si-N-Si, Si-O-Si, Si-OH and N-H bonds.

3. Method according to one of the preceding claims **characterized in that** the entity is capable, through physicochemical interactions with the dielectric layer on which it is deposited, of influencing the radiative and non-radiative electron transitions between the energy levels in the band gap caused by the external exciting electromagnetic radiation.

4. Method according to one of the preceding claims **characterized in that** the dielectric layer has the following stoichiometry of Si, N and O atoms:
SiOₓ with 0 < x < 2, Si_{y}N_{z} with 1 < y < 3 and 0 < z < 4 or SiₜOᵤNᵥ with 1 < t < 3.0 < u < 1 and 0 < v < 2.

5. Method according to one of the preceding claims **characterized in that** the silicon nanoparticles have a size in the range from 1 to 20 nm, preferably the range from 1 to 7 nm.

6. Method according to one of the preceding claims **characterized in that** the dielectric layer has a thickness of less than 500 nm, preferably from 30 to 200 nm, and preferentially from 50 to 150 nm.

7. Method according to one of the preceding claims **characterized in that** the dielectric layer is a dielectric layer of silicon nitride in which silicon nanoparticles are distributed and which is partially hydrogenated and the stoichiometry of silicon atoms and nitrogen atoms of which is SiNₓₐ with xa ranging from 0.4 to 0.8.

8. Method according to one of the preceding claims **characterized in that** the dielectric layer does not comprise metallic particles either in its mass or on the surface.

9. Method according to one of the preceding claims **characterized in that** the exciting electromagnetic radiation is a light radiation that is visible to the human eye, an infrared, ultraviolet or X-ray radiation.

10. Method according to one of the preceding claims **characterized in that** detection of the luminescence of the dielectric layer is carried out in step c) in the form of a multi-colour image.

11. Method according to one of the preceding claims **characterized in that** the dielectric layer is obtained by a plasma-enhanced chemical vapour deposition process called PECVD.

12. Method according to one of the preceding claims **characterized in that** detection is carried out without adding a luminescent agent to the entity.

13. Method according to one of the preceding claims **characterized in that** the entity is a biological entity, in particular a molecule forming part of the composition of cells, such as a protein, a lipid, a DNA, an RNA, a cell or a cell organelle such as a nucleus or a mitochondrion.

14. Method according to claim 13 **characterized in that** the entity is a cell organelle such as a nucleus or a mitochondrion, or preferably, the entity is a living cell.

15. Method according to claim 14 **characterized in that** the entity is a living cell which is deposited and grown on the dielectric layer.

16. Use of a method according to one of claims 13 to 15 to perform cell differentiation.

17. Use of a method according to one of claims 13 to 15 to diagnose a pathology, and in particular a cancer.

18. Use of a method according to one of claims 13 to 15 to evaluate the effectiveness of a treatment.
